# EUROPÄISCHE PATENTANMELDUNG

(11) **EP 3 040 020 A1**
(43) Veröffentlichungstag der Anmeldung: **06.07.2016**
(21) Anmeldenummer: 15400047.5
(22) Anmeldetag: 19.10.2015
(51) Int. Cl.: A61B 5/04, A61B 5/02, A61B 5/08, A61B 90/96, A61B 90/90, G06F 19/00, G06Q 50/22

(54) **VERFAHREN UND VORRICHTUNG ZUR EINDEUTIGEN ZUORDNUNG VON MEDIZINISCHEN MESSEINRICHTUNGEN**

(30) Priorität: 30.12.2014 DE 102014019594
(71) Anmelder: seca ag, 4153 Reinach BL 1 (CH)
(72) Erfinder: Leuner, Rüdiger, 21107 Hamburg (DE)
(74) Vertreter: Patentanwälte Klickow & Partner Partnerschaftsgesellschaft mbB

(57) **Zusammenfassung**

Die Erfindung betrifft eine Messwerteaufnahmevorrichtung und ein Verfahren zur Ermittlung und Übertragung von Patientenmesswerten an eine Anzeige- und Bedieneinheit und, oder Zentraleinheit aufweisend wenigstens einen Messwerteaufnahmesensor zur Messung von Körpereigenschaften und, oder Körperfunktionen, wobei die Messwerteaufnahmevorrichtung durch wenigstens eine Identifizierungskodierung identifizierbar ist und funktionell mit wenigstens einer Abtastungsvorrichtung zur Erfassung der Identifikationskodierung oder des Identifikationsmerkmals eines Patienten und, oder zur Erfassung der wenigstens einen Identifikationskodierung der Messwerteaufnahmevorrichtung zusammenwirkt, sodass eine fehlerreduzierte Zuordnung oder Vertauschung von Identifikationskodierung oder Identifikationsmerkmal eines Patienten und seiner gemessenen Körpereigenschaft und, oder Körperfunktion unterstützt ist.

## Beschreibung

Die Erfindung betrifft eine Messwerteaufnahmevorrichtung und ein Verfahren zur Ermittlung und Übertragung von Patientenmesswerten an eine Anzeige- und Bedieneinheit und, oder Zentraleinheit, aufweisend wenigstens einen Messwerteaufnahmesensor zur Messung von Körpereigenschaften und, oder Körperfunktionen.

Zur Messung von Körpereigenschaften und Körperfunktionen von Subjekten, z.B. menschlichen Personen oder Tieren, werden sowohl mobile als auch stationäre Messgeräte eingesetzt. Bei den zu messenden Eigenschaften oder Funktionen handelt es sich häufig um Bio- bzw. Körperimpedanzen, Herzfunktionen, Nervenfunktionen oder Elektrokardiogrammen. Weitere Messgrößen in diesem Zusammenhang können Körpergewicht, Körperzusammensetzungen, geometrische Abmessungen, Fingerabdrücke, Körpertemperaturen an verschiedenen Stellen, Zusammensetzung des verwerteten Atemgases, Atemgasvolumina, Blutdruckwerte usw. sein.

Für diese Messungen ist es erforderlich, dass das Messgerät mit dem zu messenden Subjekt verbunden wird und, oder dass die jeweiligen Messgrößen in geeigneter Weise gemessen oder erfasst und häufig auch verarbeitet und weitergeleitet werden. Ob eine Messung durch Kontakt mit dem Patienten oder kontaktlos erfolgen kann, ist abhängig von der Körpereigenschaft, der Körperfunktion bzw. dem Vitalparameter die gemessen werden soll und der dazu verwendeten Messmethode.

Die verfügbare Messtechnik generell und insbesondere in der Medizintechnik erlaubt heute, dass immer mehr Vitalparameter, verschiedene Körpereigenschaften und Körperfunktionen in immer kürzerer Zeit ermittelt und elektronisch weiterverarbeitet werden können.

Damit die Untersuchungskosten gering bleiben, sind wirtschaftlich optimierte Verarbeitungs- und Auswertungsprozesse der jeweiligen Messgrößen erforderlich. Um dieses Ziel zu erreichen, werden insbesondere bei der Anwendung in Krankenhäusern und großen Gemeinschaftspraxen, in denen sehr viele Messwerte von einer Vielzahl von Patienten aufgenommen werden müssen, nicht mehr Vorrichtungskombinationen bestehend aus einer Messwerteaufnahmevorrichtung und einer zugeordneten Messwertverarbeitungs- und Auswertevorrichtung eingesetzt, sondern es werden durch eine Vielzahl von Messwerteaufnahmevorrichtungen, die aufgenommenen Messwerte einer meist zentralen Messwertverarbeitungs- und Auswertevorrichtung zugeleitet.

Dieses Vorgehen ist eine Konsequenz aus der Tatsache, dass immer mehr Funktionalitäten von intelligenten Sensoren ohne komplizierte Anzeige- und Bedieneinheiten ausgeführt werden müssen und statt einer dezentralen Messwertverarbeitung durch einzelne Messgeräte vermehrt zentrale Auswertungs-, Anzeige- und Bedieneinheiten mit entsprechender Vernetzung eingesetzt werden. Dabei müssen die als Satelliten bezeichneten Messwerteaufnahmevorrichtungen eindeutig einem Patienten zuordbar sein.

Für eine spätere Auswertung der Messdaten ist es erforderlich, dass eine eindeutige Zuordnung sowohl des Patienten als auch der verwendeten Messeinrichtung zu einem bestimmten Datensatz erfolgt. Für die Diagnose und Festlegung der geeigneten Behandlung zur Heilung der Krankheit des jeweiligen Patienten ist eine absolut korrekte Zuordnung unabdingbar, eine fehlerhafte Datenbasis hinsichtlich Inhalt oder Zuordnung kann äußerst negative Auswirkungen nach sich ziehen.

Es kann zum einen die Situation vorliegen, dass die gleiche Messwerteaufnahmevorrichtung nacheinander an unterschiedlichen Patienten verwendet wird, darüber hinaus kann aber auch ein bestimmter Patient in einer zeitlichen Abfolge nacheinander mit unterschiedlichen Messwerteaufnahmevorrichtungen vermessen werden. Die Durchführung der einzelnen Messungen erfolgt häufig vor dem eigentlichen Behandlungsgespräch mit dem behandelnden Arzt.

In Krankenhäusern, Großpraxen und anderen Behandlungseinrichtungen mit hohem Patientenaufkommen bzw. zu vermessenden Personen ist es übliches Vorgehen, die Patienten bzw. Personen mit einer Identifikationseinrichtung zu versehen, die häufig mittels eines Armbandes festgelegt wird und eine Identifizierungskodierung enthält. Bei der Identifizierungskodierung kann es sich um eine Identifikationsnummer handeln, die dem Armband als alphanumerisches Zeichen eingeprägt ist.

Alternativ oder additiv zur Identifikationsnummer kann eine Identifikationskodierung als vorzugsweise maschinenlesbaren Barcode, als Farbenfolgekombination oder als eine andere geeignete Identifikationskodierung ausgebildet sein. Neben der Verwendung eines sogenannten Patientenarmbandes kann additiv oder alternativ als Identifikationsmerkmal eine der unverwechselbaren Eigenschaften des Körpers von Patienten genutzt werden, wie zum Beispiel der Fingerabdruck oder die geometrisch-farblichen Merkmale der Iris eines Auges.

Wird der durch die Identifikationseinrichtung identifizierbare Patient einer Vermessung bzw. Messwerteerfassung zugeführt, erfolgt die Zuordnung der Messwerte in der Regel durch das Bedienpersonal der Messvorrichtungen oder im Fall einer maschinenlesbaren, vorzugsweise optisch oder sensorisch abtastbaren Identifikationskodierung oder dem Identifikationsmerkmal durch eine entsprechende separate Messwerteaufnahmevorrichtung.

Erfolgt die Zuordnung der Messwerte durch das Bedienpersonal der Messvorrichtungen, sind Irrtümer und fehlerhafte Messwertezuordnungen, Ablesefehler oder Vertauschungen eine immer wiederkehrende Fehlerquelle.

Erfolgt die Zuordnung der Messwerte durch eine entsprechende separate Messwerteaufnahmevorrichtung, wird dies z.B. durch einen Barcodescanner oder einen Sensor realisiert, der die dann zwingend erforderliche maschinenlesbare Identifikationskodierung oder das Identifikationsmerkmal aufnimmt. Diese Methode ist zuverlässiger als die Zuordnung der Messwerte durch das Bedienpersonal der Messvorrichtungen, aber durch die Mitwirkung menschlicher Handlungen sind auch hier fehlerhafte Zuordnungen nicht vollständig ausgeschlossen. Liegt eine fehlerhafte Zuordnung vor, ist es praktisch nicht möglich, dies zu kontrollieren oder durch eine Prüfung den Zuordnungsfehler zu entdecken.

Es ist daher Aufgabe der Erfindung, ein Verfahren sowie eine Vorrichtung zur Zuordnung von Messwerten zu dem jeweils vermessenen Patienten bereitzustellen, welche die Zuverlässigkeit der Messwertezuordnung generell erhöht und die Prüfungsmöglichkeiten der Zuordnung verbessert sowie einen kostengünstigen Aufbau und die einfache Handhabung unterstützt.

Erfindungsgemäß wird diese Aufgabe gelöst durch die Identifizierbarkeit sowohl des Patienten als auch der wenigstens einen Messwerteaufnahmevorrichtung für die Messung von Körpereigenschaften und Körperfunktionen durch wenigstens eine Identifikationskodierung, wobei die Messwerteaufnahmevorrichtung funktionell mit wenigstens einer Abtastungsvorrichtung zur Erfassung der Identifikationskodierung oder des Identifikationsmerkmals eines Patienten und, oder zur Erfassung der wenigstens einen Identifikationskodierung der Messwerteaufnahmevorrichtung zusammenwirkt, sodass eine fehlerreduzierte Zuordnung oder Vertauschung von Identifikationskodierung oder Identifikationsmerkmal eines Patienten und seiner gemessenen Körpereigenschaft und, oder Körperfunktion unterstützt ist, sowie der Verknüpfung der jeweiligen Messwerte und Abtastergebnisse als Verfahren zur Messdatenhandhabung.

Die Erfindung erkennt, dass die Nachteile des Standes der Technik wenigstens reduziert werden können, indem der Satellit und oder dessen Sensoren mit der wenigstens einen Messwerteaufnahmevorrichtung für die Messung von Körpereigenschaften und Körperfunktionen mit einer Identifikationskodierung ausgestattet ist, die dessen eindeutige Identifizierung erlaubt und funktionell oder physisch kombiniert wird mit wenigstens einen Messwerteaufnahmevorrichtung für die Abtastung sowohl der Identifikationskodierung oder des Identifikationsmerkmals des Patienten aus auch für die Identifikationskodierung der wenigstens einen Messwerteaufnahmevorrichtung für die Messung von Körpereigenschaften und Körperfunktionen. Als Abtastvorrichtung eignen sich zum Beispiel Barcodescanner oder optische Erfassungsvorrichtungen wie Kameras und opto-elektronische Erfassungssysteme.

Die Identifikationskodierung oder das Identifikationsmerkmal des Patienten sowie die zu messenden jeweiligen Körpereigenschaften bzw. Körperfunktionen und, oder die Identifikationskodierung der Messwerteaufnahmevorrichtung für die Messung von Körpereigenschaften und Körperfunktionen können erfindungsgemäß zueinander gemeinsam zeitgleich oder zeitversetzt gemessen und als gemeinsames Datenpaket entweder sofort an die zentrale Bedien- und Anzeigeeinheit übertragen oder zunächst auf dem Satelliten gespeichert und für die spätere Übertragung bevorratet werden.

Durch die gemeinsame, zeitgleiche oder zeitversetzte Messung und gemeinsame Ablage bzw. Übertragung der Patientenmesswerte, der Identifikationskodierung oder des Identifikationsmerkmals des Patienten und, oder der Identifikationskodierung der vorzugsweise als Satelliten ausgebildeten Messwerteaufnahmevorrichtung für die Messung von Körpereigenschaften und Körperfunktionen wird eine fehlerhafte Zuordnung oder Vertauschung von Identifikationskodierung oder Identifikationsmerkmal und die jeweilige Körpereigenschaft bzw. Körperfunktion verhindert.

Ein weiterer besonderer Vorteil der Erfindung besteht in der eröffneten Möglichkeit, die Zuordnung kontrollieren zu können. Neben der Zuordnung der Messdaten zu einem Patienten infolge der Verknüpfung der jeweiligen Messwerte und Abtastergebnisse wird diesem Datenpaket weiterhin das Identifikationsergebnis der Messwerteaufnahmevorrichtung zugefügt, welches die Messungen durchgeführt hat. Erfindungsgemäß werden drei Informationen verknüpft: Die eigentlichen Messdaten mit den Identifizierungsdaten des identifizierten Patienten und den Identifizierungsdaten der identifizierten Messwerteaufnahmevorrichtung. Über Plausibilitätschecks, beispielsweise zeitlicher oder örtlicher Natur, oder dem für die jeweilige Messung erforderlichen Messverfahren können Zuordnungen kontrolliert werden. Es ist eindeutig nachvollziehbar, welche Messwerte bei welchem Patienten mit welcher Messwerteaufnahmevorrichtung erfasst wurden.

Durch die Anwendung der Erfindung ist es möglich, relativ einfach strukturierte Messwerteaufnahmevorrichtungen ohne implementierte gerätetechnische Intelligenz zu verwenden. Bei einer auf den Messvorgang folgenden Datenauswertung vor oder nach deren Übertragung kann unterschieden werden zwischen gültigen Messdaten und ungültigen Messdaten. Zeigt die Datenauswertung einen Fehler oder Abweichungen auf, kann die sofortige Wiederholungsmessung initiiert werden.

Bei der Durchführung eines Identifikationsvorganges kann bevorzugt die gleiche Abtastvorrichtung sowohl für die Abtastung der Identifikationskodierung der Messwerteaufnahmevorrichtung als auch für die Identifikationskodierung oder des Identifikationsmerkmals des Patienten verwendet werden, sofern beide eine gleichgeartete Abtastung ermöglichen. Ist beispielsweise die Identifikationskodierung der Messwerteaufnahmevorrichtung und auch die Identifikationskodierung oder des Identifikationsmerkmal des Patienten als ein optisch abtastbarer Barcode realisiert, kann ein Barcodescanner für beide Identifizierungsvorgänge genutzt werden.

Um die fehlerfreie Messwertezuordnung zu unterstützen, sieht die Erfindung mehrere Arbeitsschritte in ggf. unterschiedlicher Reihenfolge vor: Im ersten Schritt wird die Identifikationskodierung der Messwerteaufnahmevorrichtung abgetastet bzw. ermittelt. Anschließend wird im zweiten Schritt gleichzeitig oder zeitversetzt die Identifikationskodierung des Patienten abgetastet und seine Messwerte werden aufgenommen. Im Anschluss daran wird durch Schritt 3 erneut die Identifikationskodierung der Messwerteaufnahmevorrichtung abgetastet bzw. ermittelt.

Ergebnis dieses Prozesses sind drei Dateninformationspakete bestehend aus der Identifikationsinformation jeweils für die Messwerteaufnahmevorrichtung und für den Patienten sowie die Messwerte in Form von Vitalparametern und, oder verschiedenen Körpereigenschaften und, oder Körperfunktionen. Die Dateninformationspakete können bereits vorder Übertragung an die Anzeige- und Bedieneinheit oder Zentraleinheit innerhalb der Messwerteaufnahmevorrichtung, die häufig als Satellit ausgebildet ist, und, oder der Abtastvorrichtung bzw. dem wenigstens einen Sensor verknüpft werden.

Die Erfindung unterstützt sowohl die simultane, d.h. unmittelbare zeitgleiche Übertragung der Messgrößen und, oder die Abtastungsergebnisse an eine Anzeige- und Bedieneinheit bzw. Zentraleinheit als auch eine zeitversetzte Übertragung. Für die zeitversetzte Übertragung der Messgrößen von Körpereigenschaften und Körperfunktionen und, oder die Abtastungsergebnisse in Form von Identifikationskodierung und, oder Identifikationsmerkmal wird eine geeignete Datenspeicherungseinheit in dem Satelliten vorgesehen.

Der Datentransfer zwischen Satellit und Anzeige- und Bedieneinheit bzw. Zentraleinheit kann auf verschiedenen leitungsbasierenden oder drahtlosen Verfahren beruhen, beispielsweise elektrisch, optisch, Funk, WLAN, Netzwerk, Bluetooth usw.

Die Erfindung wird nachfolgend mit Bezug auf die Figuren erläutert. Im Einzelnen zeigen
- Fig. 1: ein Beispiel einer Abtastvorrichtung (30) in Form eines kompakten Moduls zum Scannen von Barcodes,
- Fig. 2: ein Beispiel einer Abtastvorrichtung (30) in Form eines kompakten Kamera-Moduls,
- Fig. 3: beispielhaft das erfindungsgemäße Verfahrenskonzept bestehend aus einer Anzeige- und Bedieneinheit (10) und davon abgesetzten, d.h. physisch getrennten Messelektronik in Form eines Satelliten (20),
- Fig. 4: beispielhaft das Konzept eines Satelliten (20) ausgebildet als Messwerteaufnahmevorrichtung für die Messung von Körpereigenschaften und Körperfunktionen sowie der Abtastung der Identifikationskodierung oder des Identifikationsmerkmals bestehend im Wesentlichen aus einer Messmatte (21) aufweisend weinigstens einen Sensor (22) zur Messung von Körpereigenschaften und Körperfunktionen und einer Abtastungsvorrichtung (30) zur Erfassung der Identifikationskodierung (40) der Messwerteaufnahmevorrichtung und der Identifikationskodierung oder des Identifikationsmerkmals des Patienten.

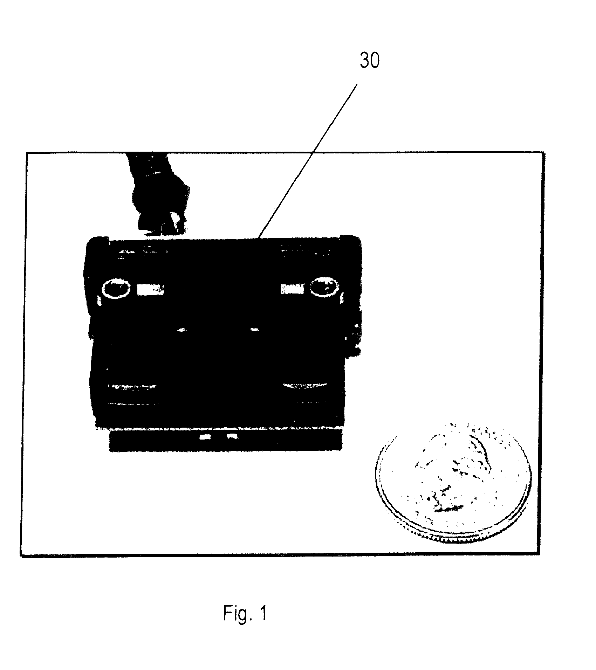

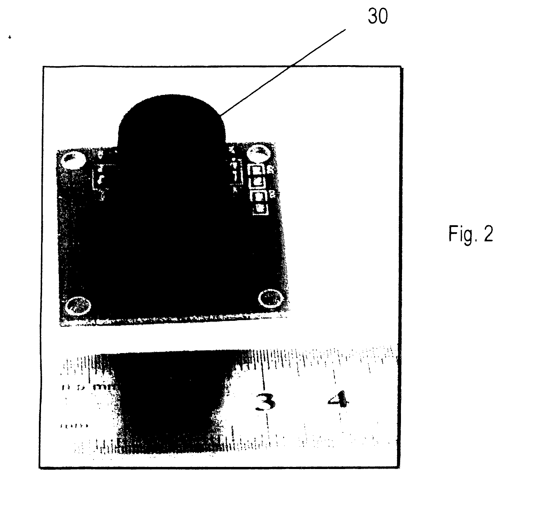

Figur 1 und 2 zeigen exemplarisch mögliche Ausgestaltungen der Abtastungsvorrichtung (30) zur Erfassung der Identifikationskodierung oder des Identifikationsmerkmals in Form von Kompaktmodulen basierend auf optischen Wirkprinzipen.

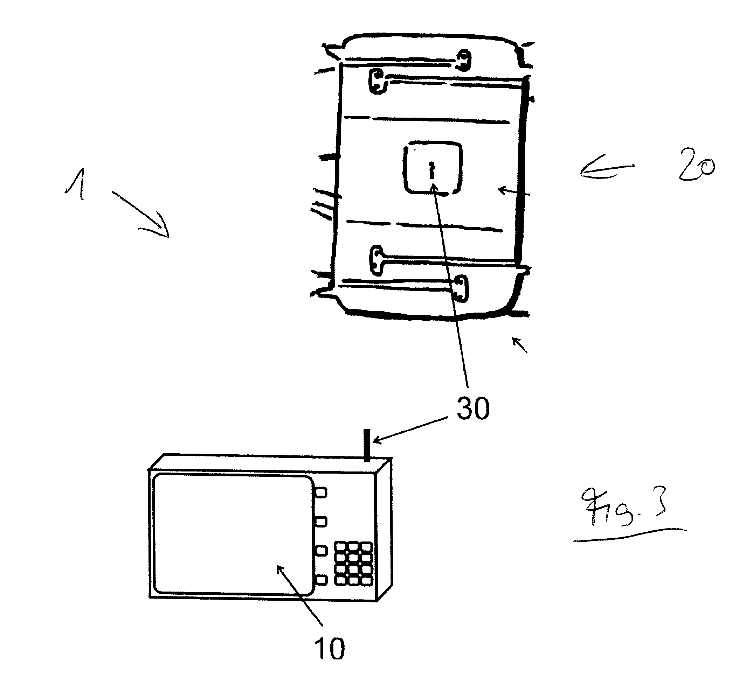

Figur 3 zeigt beispielhaft das Konzept bestehend aus einer Anzeige- und Bedieneinheit (10) und davon abgesetzten, d.h. physisch getrennten Messwerteaufnahmevorrichtung in Form eines Satelliten (20). Mit diesem Konzept wird der erforderliche, wirtschaftlich optimierte Verarbeitungs- und Auswertungsprozess der jeweiligen Messgrößen erreicht. Insbesondere in der Anwendung im Rahmen sehr vieler aufzunehmender Messwerte eines oder einer Vielzahl von Patienten können auf diese Weise und mittels einer Vielzahl von Satelliten (20) die aufgenommenen Messwerte einer meist zentralen Messwertverarbeitungs- und Auswertevorrichtung (10) zugeleitet werden.

Durch dieses Konzept wird der Tatsache Rechnung getragen, dass immer mehr Funktionalitäten von intelligenten Sensoren ohne komplizierte Anzeige- und Bedieneinheiten ausgeführt werden müssen und statt einer dezentralen Messwertverarbeitung durch einzelne Messgeräte vermehrt zentrale Auswertungs-, Anzeige- und Bedieneinheiten mit entsprechender Vernetzung eingesetzt werden.

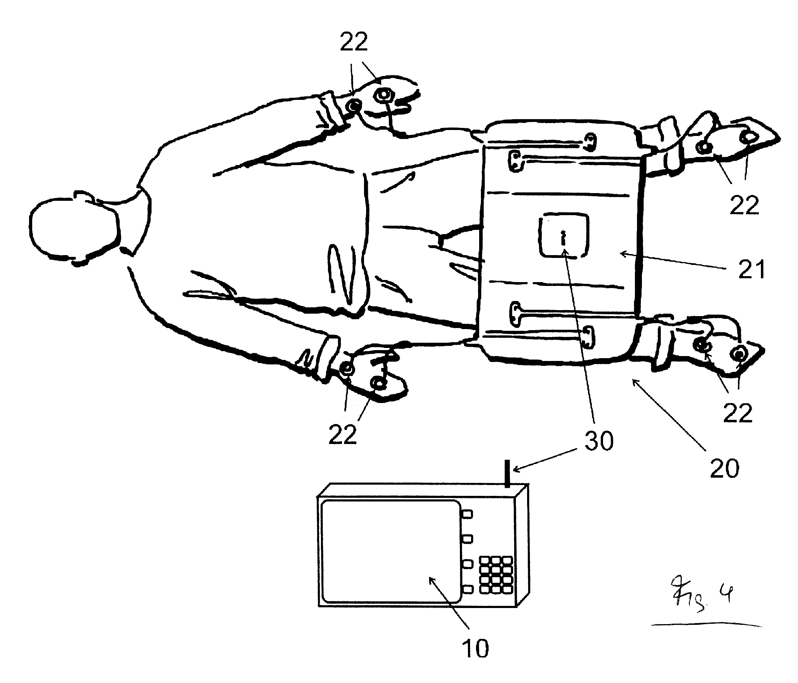

Figur 4 illustriert beispielhaft das erfindungsgemäße Konzept eines Satelliten (20) ausgebildet als Messwerteaufnahmevorrichtung für die Messung von Körpereigenschaften und Körperfunktionen sowie der Abtastung der Identifikationskodierung oder des Identifikationsmerkmals bestehend im Wesentlichen aus einer Messmatte (21) aufweisend wenigstens einen Sensor (22) zur Messung von Körpereigenschaften und Körperfunktionen und einer Abtastungsvorrichtung (30) zur Erfassung der Identifikationskodierung oder des Identifikationsmerkmals des Patienten und, oder der der Messwerteaufnahmevorrichtung (20).

Üblicherweise ist eine Abtastungsvorrichtung (30) zur Erfassung der Identifikationskodierung oder des Identifikationsmerkmals pro Satellit (20) ausreichend, Figur 5 zeigt verschiedene Positionierungsmöglichkeiten auf. Die Abtastungsvorrichtung (30) ist im gezeigten Beispiel im Bereich der Messmatte (21) und, oder im Bereich wenigstens eines Sensors (22) angeordnet und damit Bestandteil der Messwerteaufnahmevorrichtung (20).

Soll die Abtastvorrichtung (30) sowohl die Identifikationskodierung oder das Identifikationsmerkmal des Patienten und die Identifikationskodierung der Messwerteaufnahmevorrichtung (20) abtasten muss die Anordnung entsprechend derart erfolgen, dass dies unterstützt ist. Eine andere Lösungsmöglichkeit besteht in einer unabhängigen und damit separaten Abtastungsvorrichtung (30).

Der Satellit (20) als Messwerteaufnahmevorrichtung kann auf verschiedenen Vorrichtungen zur Messung von Körpereigenschaften und Körperfunktionen basieren, die mit wenigstens einer Vorrichtung zur Abtastung (30) der Identifikationskodierung oder des Identifikationsmerkmals des Patienten und, oder der Identifikationskodierung der Messwerteaufnahmevorrichtung (20) kombiniert werden kann.

Die in Figur 5 beispielhaft gezeigte Messmatte (21) als Basis für den Satelliten (20) bietet Handhabungsvorteile. Da die Messmatte (21) während der Messwertaufnahme quer über den Beinen des Patienten liegt, sind die Enden der Messmatte (21) mit ihrer bevorzugten Positionierung wenigstes einer Abtastungsvorrichtung (30) zur Erfassung der Identifikationskodierung oder des Identifikationsmerkmals des Patienten sehr leicht durch den Patienten erreichbar. Ein Einscannen der Identifikationskodierung von dem Patientenarmband oder die Erfassung eines Identifikationsmerkmals, zum Beispiel der optischen Erfassung der Iris, ist in einfacher Weise und insbesondere unter aktiver Mithilfe des Patienten möglich.

Weiterhin zeigt Figur 4 anhand verschiedener Positionierungsbeispiele in additiver oder alternativer Form unterschiedliche Möglichkeiten auf, die Identifikationskodierung (40) der Messwerteaufnahmevorrichtung (20) anzuordnen. Denkbare Positionen sind an geeigneter Stelle auf oder in der Messmatte (21) und, oder auf wenigstens einem der Sensoren (22). Wird wenigstens eine Identifikationskodierung (40) auf einem Sensor (22) vorgesehen kann zusätzlich zu der Zuordnung des gemessenen Wertes zu einer Messwerteaufnahmevorrichtung (20) auch eine Zuordnung zu einem einzelnen Sensor (22) vorgenommen werden.

Das Verfahren kann insbesondere durch die folgenden Verfahrensschritte optimal weitergebildet werden.

Insbesondere ist vorgesehen, dass ein Schritt e. ausgeführt wird, der im Wesentlichen dem Schritt d. gleicht, sodass die Erfassung der Identifikationskodierung (40) der Messwertevornahmevorrichtung (20) zweimal erfolgt.

Insbesondere ist vorgesehen, dass der Schritt e nach den Schritten a-d ausgeführt wird.

Insbesondere ist vorgesehen, dass die Schritte a-d zeitgleich ausgeführt werden.

Insbesondere ist vorgesehen, dass wenigstens einer der Schritte a, b zeitversetzt zu den Schritten d und, oder e ausgeführt wird, wobei die wenigstens eine Datenspeichervorrichtung der Messwerteaufnahmevorrichtung (20) die Messwerte der Sensoren (22) und, oder der wenigstens einen Abtastungsvorrichtung (30) temporär oder dauerhaft speichert.

Insbesondere ist vorgesehen, dass die Schritte a und b vertauscht sind.

Insbesondere ist vorgesehen, dass die Erfassungsschritte b und d, e durch eine optische oder opto-elektronische Abtastung realisiert werden, sodass eine Abtastvorrichtung (30) für alle Erfassungsschritte einsetzbar ist.

Insbesondere ist vorgesehen, dass die Messwerteergebnisse aus Schritt a und die Erfassungsergebnisse aus Schritt b miteinander verknüpft werden.

Insbesondere ist vorgesehen, dass die verknüpften Ergebnisse von Schritt a und b mit den Erfassungsergebnissen von Schritt d verknüpft werden.

Insbesondere ist vorgesehen, dass die Zuordnung der Ergebnisse von Schritt a, b und d durch Verknüpfung durch die Erfassungsergebnisse von Schritt e kontrollierbar sind dadurch, dass die durch Schritt d und e erfasste Identifikationskodierung (40) identisch sein muss.

Insbesondere ist vorgesehen, dass die Zuordnungskontrolle durch einen Plausibilitätscheck in zeitlicher oder örtlicher Hinsicht erfolgt.

Insbesondere ist vorgesehen, dass wenigstens einer der Erfassungs- oder Messschritte simultan zu Schritt c. erfolgt.

Insbesondere ist vorgesehen, dass Schritt c. durch ein drahtloses Übertragungsverfahren realisiert ist.

## Patentansprüche

1. Messwerteaufnahmevorrichtung (20) zur Ermittlung und Übertragung von Patientenmesswerten an eine Anzeige- und Bedieneinheit und, oder Zentraleinheit (10), aufweisend wenigstens einen Messwerteaufnahmesensor (22) zur Messung von Körpereigenschaften und, oder Körperfunktionen, **dadurch gekennzeichnet, dass** die Messwerteaufnahmevorrichtung (20) durch wenigstens eine Identifizierungskodierung (40) identifizierbar ist, wobei die Messwerteaufnahmevorrichtung (20) funktionell mit wenigstens einer Abtastungsvorrichtung (30) zur Erfassung der Identifikationskodierung oder des Identifikationsmerkmals eines Patienten und, oder zur Erfassung der wenigstens einen Identifikationskodierung der Messwerteaufnahmevorrichtung (20) zusammenwirkt, sodass eine fehlerreduzierte Zuordnung oder Vertauschung von Identifikationskodierung oder Identifikationsmerkmal eines Patienten und seiner gemessenen Körpereigenschaft und, oder Körperfunktion unterstützt ist.

2. Messwerteaufnahmevorrichtung (20) nach Anspruch 1, **dadurch gekennzeichnet, dass** die Identifikationskodierung (40) der Messwerteaufnahmevorrichtung (20) derart ausgestaltet ist, dass die Identifikationskodierung (40) der Messwerteaufnahmevorrichtung (20) und die Identifikationskodierung oder das Identifikationsmerkmal des Patienten durch wenigstens eine Abtastungsvorrichtung (30) abtastbar sind.

3. Messwerteaufnahmevorrichtung (20) nach Anspruch 1, **dadurch gekennzeichnet, dass** die wenigstens eine Abtastungsvorrichtung (30) auf einem optischen oder opto-elektronischen Wirkprinzip basiert.

4. Messwerteaufnahmevorrichtung (20) nach Anspruch 1, **dadurch gekennzeichnet, dass** die Identifikationskodierung (40) der Messwerteaufnahmevorrichtung (20) ein Barcode ist.

5. Messwerteaufnahmevorrichtung (20) nach Anspruch 1, **dadurch gekennzeichnet, dass** die Messwerteaufnahmevorrichtung (20) als Satellit ausgebildet ist, sodass die autarke Messung von Körpereigenschaften und, oder Körperfunktionen und, oder die Erfassung der Identifikationskodierung (40) der Messwerteaufnahmevorrichtung (20) und, oder die Erfassung der Identifikationskodierung oder des Identifikationsmerkmals des Patienten unterstützt ist.

6. Messwerteaufnahmevorrichtung (20) nach Anspruch 1, **dadurch gekennzeichnet, dass** die Messwerteaufnahmevorrichtung (20) zur Messung von Körpereigenschaften und, oder Körperfunktionen eines Patienten und, oder die wenigstens eine Abtastungsvorrichtung (30) in eine Messmatte (21) integriert ist.

7. Messwerteaufnahmevorrichtung (20) nach Anspruch 6, **dadurch gekennzeichnet, dass** die wenigstens eine Abtastungsvorrichtung (30) derart in der Messmatte (21) positioniert ist, dass sowohl die Identifikationskodierung (40) der Messwerteaufnahmevorrichtung (20) als auch die Identifikationskodierung oder das Identifikationsmerkmal des Patienten abtastbar sind.

8. Messwerteaufnahmevorrichtung (20) nach Anspruch 1, **dadurch gekennzeichnet, dass** die wenigstens eine Abtastungsvorrichtung (30) als optisch oder opto-elektronisch arbeitendes Modul ausgebildet ist.

9. Messwerteaufnahmevorrichtung (20) nach Anspruch 1, **dadurch gekennzeichnet, dass** die wenigstens eine Abtastungsvorrichtung (30) ein Barcodescanner oder eine Kamera ist.

10. Messwerteaufnahmevorrichtung (20) nach Anspruch 1, **dadurch gekennzeichnet, dass** wenigstens eine Datenspeichervorrichtung zur temporären oder dauerhaften Speicherung von Messwerten der Messwerteaufnahmevorrichtung (20) und, oder der wenigstens einen Abtastungsvorrichtung (30) vorgesehen ist.

11. Messwerteaufnahmevorrichtung (20) nach Anspruch 1, **dadurch gekennzeichnet, dass** eine Drahtlosübertragungseinrichtung für die Kommunikation und, oder Datenübertragung zwischen der wenigstens einen Messwerteaufnahmevorrichtung (20) und der Auswertungs-, Anzeige- und, öder Bedieneinheit (10) vorgesehen ist.

12. Messwerteaufnahmevorrichtung (20) nach Anspruch 11, **dadurch gekennzeichnet, dass** die Drahtlosübertragungseinrichtung durch eine Infrarot- oder WLAN- oder Bluetoothschnittstelle gebildet ist.

13. Vermessungssystem (1) zur Ermittlung und Auswertung von Messdaten bestehend aus Körpereigenschaften, Körperfunktionen und, oder Vitalparametern eines Patienten aufweisend wenigstens eine Messwerteaufnahmevorrichtung (20) zur Messung von Messdaten nach einem der Ansprüche 1 bis 12 und wenigstens einer Auswertungs-, Anzeige- und, oder Bedieneinheit (10) zur Auswertung und Weiterverarbeitung der Messdaten.

14. Verfahren zur Ermittlung und Übertragung von Patientenmesswerten an eine Anzeige- und Bedieneinheit oder Zentraleinheit (10) aufweisend die Schritte
a. Messen der Körpereigenschaften und, oder Körperfunktionen eines Patienten,
b. Erfassung der Identifikationskodierung und, oder des Identifikationsmerkmals des zu vermessenden Patienten,
c. Übertragung der gemessenen und erfassten Daten an eine Anzeige- und Bedieneinheit oder Zentraleinheit, **gekennzeichnet durch**
Schritt d. Erfassung der Identifikationskodierung (40) der Messwerteaufnahmevorrichtung (20), wobei die gemessenen und, oder erfassten Dateninformationspakete zu einem Datenpaket verknüpft werden, sodass eine fehlerreduzierte Zuordnung oder Vertauschung von Identifikationskodierung oder Identifikationsmerkmal und der jeweiligen Körpereigenschaft und, oder Körperfunktion unterstützt wird.

15. Verfahren zur Ermittlung und Übertragung von Patientenmesswerten an eine Anzeige- und Bedieneinheit oder Zentraleinheit (10) nach Anspruch 14, **dadurch gekennzeichnet, dass** Schritt d. als erster Schritt ausgeführt wird und den Schritten a-c vorangestellt ist.
